# EUROPEAN PATENT SPECIFICATION

(11) **EP 3 996 597 B1**
(45) Date of publication and mention of the grant of the patent: **28.02.2024**
(21) Application number: 20754302.6
(22) Date of filing: 10.07.2020
(51) Int. Cl.: A61B 6/10, A61B 6/00, G03B 42/04, G03C 3/00, A61B 6/04

(54) **DIGITALIZATION ARRANGEMENT FOR AN ANALOG MAMMOGRAPHY APPARATUS FOR CONTINUOUS OPERATION**
DIGITALISIERUNGSANORDNUNG FÜR EIN ANALOGES MAMMOGRAFIEGERÄT ZUM KONTINUIERLICHEN BETRIEB
AGENCEMENT DE NUMÉRISATION POUR UN APPAREIL DE MAMMOGRAPHIE ANALOGIQUE POUR FONCTIONNEMENT CONTINU

(30) Priority: 10.07.2019 FI 20195625
(43) Date of publication of application: 18.05.2022
(73) Proprietor: Innomentarium Oy, 90590 Oulu (FI)
(72) Inventor: IHME, Jouni, 90940 Jääli (FI)
(74) Representative: Berggren Oy
(86) International application number: PCT/FI2020/050493
(87) International publication number: WO 2021/005273

(56) References cited:
- EP-A1- 0 775 467
- US-A1- 2012 093 295

## Description

### Field of the invention

The present invention relates to mammographic medical measurements and respective mammogram machines (i.e. mammographic apparatuses), which have traditionally been implemented as analog devices.

### Background of the invention

Mammography is a technique for detecting possible breast tumors and breast cancer among women. Mammography has been implemented in an organized manner by society by inviting women to attend regular screenings of their breasts in e.g. their local health center. Mammographic measurements are performed by a mammogram machine, which apply low-energy X-rays into human breasts, and received images are then analyzed for abnormal findings.

US 7,869,575 ("Brandstatter") discloses a basic implementation of an X-ray examination system for analog mammography, which comprises an X-ray film (in a film cassette) being exposed to an X-ray beam providing examination images of a female patient. The system comprises additionally a labeling device which can be set as a movable element in view to the X-ray film. The labelling device includes an exposure beam for exposing the X-ray film with patient-specific information. A movable screen element shields the labeling device from the X-rays during the actual X-ray examination.

WO 2004/058063 ("Siltanen / Instrumentarium") discloses a mammography apparatus for advanced detection of breast cancer. The device has an X-ray source and an analog X-ray detector for receiving X-ray irradiations from at least two different directions. Furthermore, Siltanen applies 3D impedance tomographic information obtained by transmitting electric energy and measuring it by an electricity meter. The processing unit compiles these two kinds of images for finding more easily the suspicious parts in the breast tissue.

US 6,533,453 ("Heidsieck") concentrates on the cassette placement within a radiography apparatus such as in mammogram machines. The inserting of the cassette into the device can be recognized and also the cassette type can be recognized. In an embodiment, the casing i.e. the housing for the cassette has cut edges so that only cassettes with bevelled corners will fit inside the apparatus. Magnetic or mechanic relay can be used in the identification of the cassette into its place. A lock for immobilization can be used in the support for the cassette, and a locking detector as an electrical contact connecting to the support is used where the locking detector signal is digitized to send the locking data into the processor. No LEDs are used in Heidsieck.

US 7,429,737 ("Wojcik") discloses a digital radiography detector that can be retrofit into an existing film-based mammography exposure system. Wojcik solves the problems in analog mammography apparatuses, such as the delay in obtaining the diagnostic image, a requirement of chemical or thermal processing, and the difficulty in providing the resulting image outside the immediate medical facility. The solution is based on two housings, an X-ray converter coupled with a detection array, readout electronics, X-ray shielding in the 1st housing, and a power source and control electronics in the 2nd housing. Wojcik does not tackle the problems arising in such digital devices after the transformation from an analog device.

The problem of mammogram apparatuses according to prior art is discussed next. The analog mammography device comprises an analog film or analog image plate which acts as a detecting element which faces the exposure of the X-ray radiation transmitted from an X-ray source and propagated thereafter through the human tissue. There is a mechanism in traditional analog mammography devices, which prevents the X-ray exposure of the analog film or analog image plate twice. Two or more X-ray exposures for the same analog film or image plate would ruin the initially detected image, and the resulting "cumulative" (i.e. double or more exposed) image would be useless for any diagnostic purposes. Analog mammography devices apply a control logic, which ensures that the analog film cassette or the image plate has been physically removed from the housing of the device, and replaced by a new, non-exposed film or image plate. The element holding the film or image plate is a bucky. The logic may apply a mechanic microswitch, or the logic may apply optical means for ensuring the change of the analog image plate.

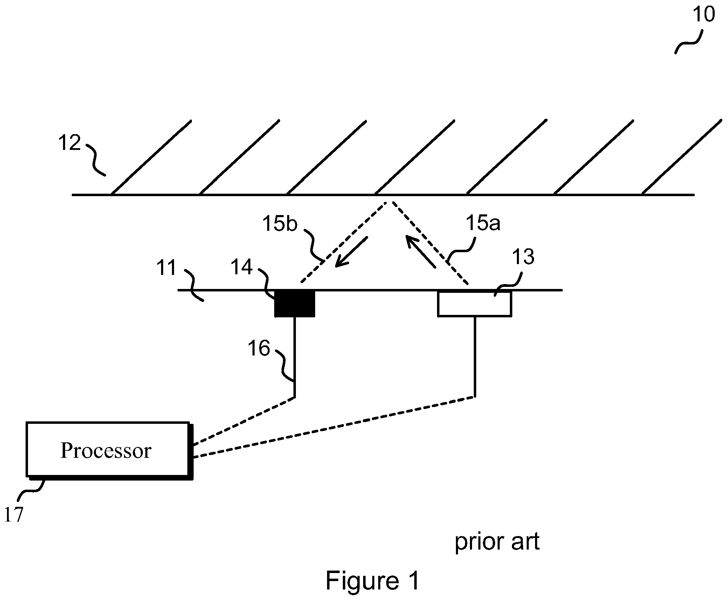

Figure 1 illustrates the situation and the principle of the above control logic applied in current analog mammogram devices 10 manufactured by Instrumentarium Corporation. The optical means used in mammogram devices manufactured by Instrumentarium comprises a light source 13 fixed near the top surface of the internal housing 11 of the device. The light source 13 emits continuous light. The analog image plate 12 has essentially a white surface, meaning that its reflectivity for visible light is good. Close by the light source 13, in the same upper surface of the internal housing, there is a photodiode or a phototransistor, i.e. a photodetector 14, which acts as light detecting means. When the analog image plate 12 is in its place within the bucky, the light 15a from the light source will reflect from the white surface of the image plate, and the reflected light 15b propagates into the photodetector 14. The photodetector output signal 16 is fed to the processor 17, which interprets the signal and decides that the analog image plate is in its place.

On the other hand, when the image plate 12 has been removed from the bucky, the reflective white surface is not there anymore. Although the light source 13 sends continuous light all the time, the reflection does not happen, and the photodetector 14 receives no light (there is no ambient or natural light in the gap between the internal housing 11 and the analog image plate 12). This signal, i.e. the photodetector output signal 16 is fed to the processor 17, which then interprets that the analog image plate 12 (or film) has been removed.

When a new analog image plate is installed into the bucky, it means that there is a reflective surface again in the path of light 15a emerging from the light source 13. The photodetector 14 senses the reflected light 15b, and the processor 17 notices and decides that there is a brand new image plate (or film) in its place in the bucky. The processor 17 then gives the X-ray source a permission to start with X-ray exposure of the human tissue resulting in a new X-ray image in the new image plate. This is the optical arrangement used in analog mammogram devices 10 manufactured by Instrumentarium Corporation in prior art.

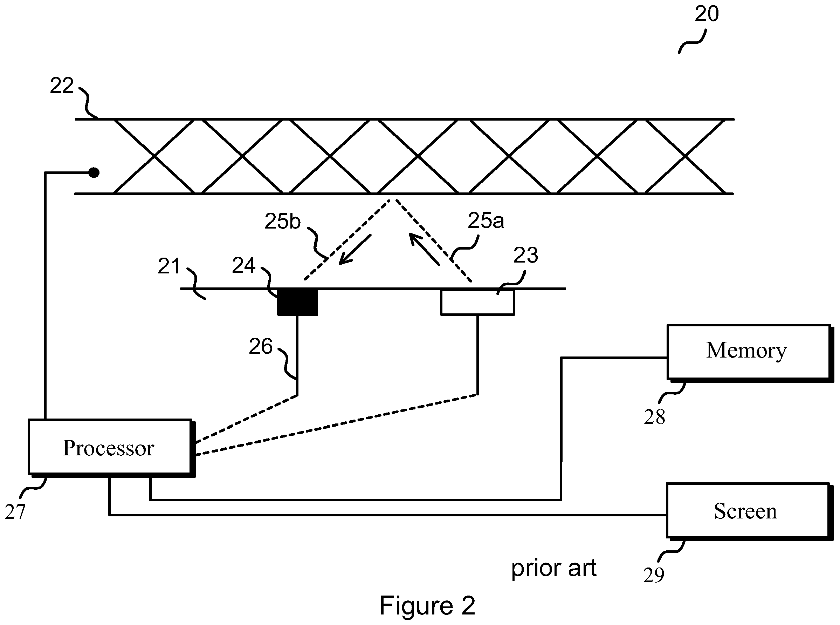

It is possible to modify the analog mammogram device (i.e. apparatus) into a digital mammogram device (i.e. apparatus) by the following alteration step. Such an altered version of the device is shown in Figure 2. The analog film or the image plate must be first removed and thereafter, replaced by a direct digital detector 22, which has in practice the same size and thus, dimensions with the traditional analog image plates or film. Thus, the direct digital detector 22 can be placed and fit into the very same bucky as before, in the otherwise analog mammogram device 20. A processor 27 needs to be configured in the altered mammogram device 20 so that the processor 27 is capable to control the X-ray exposure process, and the processing of the obtained digital image in the direct digital detector 22. The direct digital detector 22 thus allows the apparatus to take a digital image with the direct digital detector straight away, which allows for direct digital transfer of the taken image to an appropriate data storage means, i.e. a memory 28, for further presentation or analysis, such as saving it as a digital image to the memory of the mammogram apparatus, to a memory of another computer or server, or generally, to a predefined location in the cloud. The presentation of the obtained image to a medical professional or any other desired person can be arranged via a screen 29. Other elements of Figure 2 conform with the elements already shown in Figure 1; namely the internal housing 21 of the device comprising the original continuous light source 23 and the original photodetector 24, and where the photodetector output signal 26 is fed to the processor 27, and where the processor 27 controls the continuous LED light source 23 to be on all the time when the apparatus 20 is switched on and ready to image. The emitted continuous light from the light source 23 is marked as 25a, and the reflected light from the lower surface of the direct digital detector 22 is marked as 25b.

As a comparison in prior art, the analog film must be processed or developed in traditional film processing means for further use, and the analog image plate requires computed radiography (CR), which means that the CR image plate must be separately scanned into a digital form after taking the analog picture.

Back to the traditional analog mammogram device applied with the direct digital detector, there is now no need to remove the direct digital detector from the device, but instead, it is now a fixed, integral element of the mammogram apparatus now transformed into a digital device. The prior art means with the light source and the photodiode/phototransistor now allow only a single X-ray exposure and image taken with the now digital apparatus. This problem now could be solved by removing the direct digital detector from the apparatus after each taken image and by installing it back into its place, but this is a complex manual procedure, and it may possibly even break the rather expensive direct digital detector during its movements. This is not a good solution to perform.

In another piece of prior art, it is in principle possible to install a separate switch arrangement, which is required to be soldered onto a circuit board integrally within the mammogram apparatus itself. This operation has a major defect in that it touches the original design of the mammogram apparatus, which is tightly regulated because of its medical characteristics and the use of X-rays onto human tissues. This is not allowed because it breaks the rights of the original manufacturer of the analog mammogram device through violation of the original design. Also technical support of a such transformed apparatus can well be cut off. This is not a feasible solution into this problem.

Therefore, as a summary from the above, the digitally transformed analog mammogram apparatus still has a functionality, which allows only a single X-ray image to be taken, and which problem has not been solved anywhere in prior art in a feasible manner.

### Summary of the invention

The scope of protection sought for various embodiments of the invention is set out by the independent claims.

The embodiments and features, if any, described in this specification that do not fall under the scope of the independent claims are to be interpreted as examples useful for understanding various embodiments of the invention.

The present invention introduces an arrangement for functionally updating a digitalized, initially analog mammography apparatus, wherein the initially analog mammography apparatus comprises a direct digital detector instead of an analog image plate or film. The arrangement is characterized in that it comprises:
- a light blocking element configured on top of a LED light source initially part of the analog mammography apparatus,
- an external pulsed light source, which is controlled to emit pulsed light, wherein the external pulsed light source is placed between an internal housing surface of the apparatus and a surface of the direct digital detector, wherein
- an original photodetector of the apparatus is able to detect a lowering or rising edge of the received light signal originated from the external pulsed light source after each taken X-ray exposure image from a patient.

In an embodiment of the invention, the arrangement further comprises:
- the external pulsed light source placed as part of an added pulsed light element, which is placable as a strip-formed element between the internal housing surface of the apparatus and the surface of the direct digital detector.

In an embodiment of the invention, the arrangement further comprises:
- the external pulsed light source locating substantially at the far end of the strip.

In an embodiment of the invention, the arrangement further comprises:
- an external controller controlling the external pulsed light source to emit pulsed light in a predetermined pulse length or frequency.

In an embodiment of the invention, the arrangement further comprises:
- the external pulsed light source or the added pulsed light element comprising an integrated controller for controlling the external pulsed light source to emit pulsed light in a predetermined pulse length or frequency.

In an embodiment of the invention, the arrangement further comprises:
- a reflective surface part placeable on top of the direct digital detector, which reflective surface part is capable to reflect light originating from the external pulsed light source towards the photodetector.

In an embodiment of the invention, the reflective surface part is a white adherable tab placed approximately in the same area where the initial LED light source light would reflect at during its propagational path to the photodetector during the use of the analog apparatus.

According to another aspect of the present invention, there is introduced a method for furnishing an analog mammography apparatus for resulting as a continuously operable digitized mammography apparatus. The method is characterized in that it comprises the steps of:
- providing a top side of a transparent section of an internal housing on top of the LED light source with a light blocking element,
- inserting an added element between the internal housing and a direct digital detector, which added element comprises an external pulsed light source,
- controlling the added, external pulsed light source with an internal or external controller to emit light pulses in a predetermined pulse length or frequency.

In an embodiment of the invention, before the providing step, the method comprises the step of:
- removing an analog image plate or film from a bucky of the analog mammography apparatus, and placing the direct digital detector into the bucky of the analog mammography apparatus.

In an embodiment of the invention, the method further comprises the step of:
- inserting and adhering a reflective surface part on top of the direct digital detector, which reflective surface part is capable to reflect light originating from the external pulsed light source towards the photodetector.

In an embodiment of the invention, the method further comprises the step of:
- turning on the external pulsed light source by the controller, by constantly emitting pulsed light towards the photodetector during the operation of the X-ray imaging.

In an embodiment of the invention, the method further comprises the step of:
- providing an external UI control means in connection to the external pulsed light source, allowing a professional user of the digitized mammography apparatus to provide a signal through the UI control means after each taken X-ray exposure image.

In an embodiment of the invention, the external UI control means is a manual button or switch.

### Brief description of the drawings

Figure 1 shows the prior art situation now in analog mammogram apparatuses applying an optical principle for monitoring the presence of an analog image plate or film,
Figure 2 illustrates the altered (i.e. analog to digital) mammogram device with a direct digital detector,
Figure 3 illustrates the arrangement according to an embodiment of the invention applied to a digitally altered analog mammogram apparatus, and
Figure 4 illustrates process steps in a flow chart showing steps for furnishing the digitally altered mammogram device according to an embodiment of the invention for obtaining the desired repeatability function for the taken X-ray images.

### Detailed description of the invention

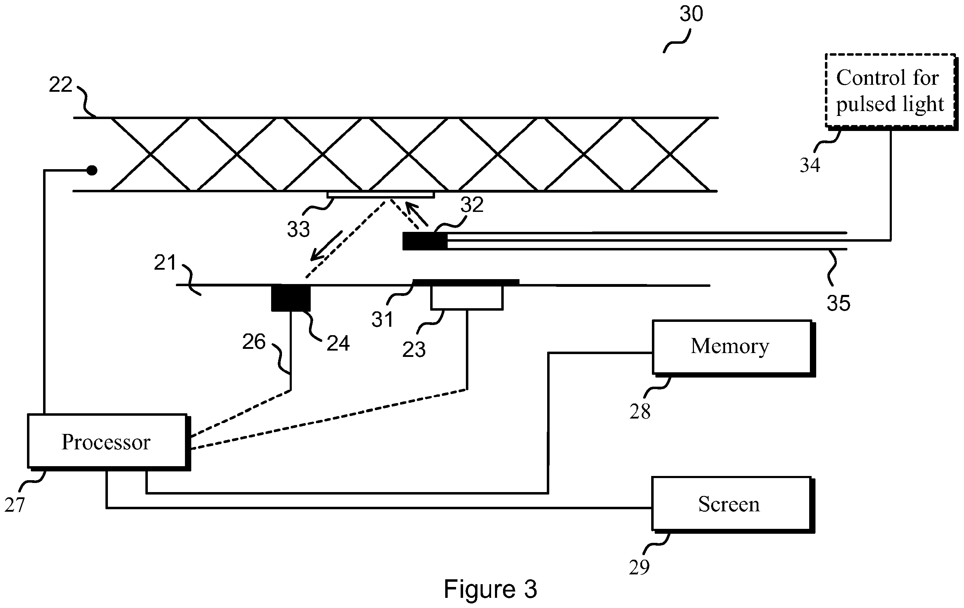

The starting point of the present invention is exemplified by Figure 3, where there has been shown the situation, where an analog mammogram device has been altered into a digital mammogram device 30 without any other alterations. In other words, the main components of the altered mammogram device 30 are the internal housing 21 of the device comprising the original continuous light source 23 and the original photodetector 24 (e.g. a photodiode or a phototransistor), and where the photodetector output signal 26 is fed to the processor 27, and where the processor 27 controls the continuous LED light source 23 to be on all the time, when the apparatus 30 is switched on and ready to image. The direct digital detector 22 is placed into the bucky, and the processor 27 is able to control both the X-ray exposure by controlling the X-ray source, and the reception of the obtained X-ray image from the direct digital detector 22. The processor 27 is able to save the obtained X-ray image to a memory 28 either locally or remotely, and possibly to show the obtained X-ray image to a medical professional or other desired person via a screen 29. The X-ray source is a relevant functional part of the mammogram apparatus as such, but for simplicity reasons, this part is not shown in Figure 3. Also the position of the imaged human tissue (i.e. a female breast) is not shown in Figure 3 for simplicity reasons.

Next the parts forming the present invention in an embodiment of it, are discussed. At a first step, the original LED light emitting continuous light, need to be taken out of the arrangement in a practical manner, which does not require any physical intervention to the original device as such. The LED light 23 locates under a small transparent section on top of the internal housing 21 of the device. In the present invention, the top side of the transparent section on top of the LED light source 23 is provided with a black adherable tab 31. In an embodiment, the tab 31 is a piece of adhesive tape in a dark colour, preferably black. In general, when considering the function of this added part, we may call the black adherable tab generally as "a light blocking element" 31 for the original LED light source 23 of the analog device.

The present invention further comprises an added pulsed light element 35, which can be inserted as a separate strip-formed element between the internal housing 21 of the device and the direct digital detector 22. Although Figure 3 shows the added pulsed light element 35 being in a kind of floating position between the two elements, the added pulsed light element 35 can well be rest right on top of the internal housing 21.

The main elements of the added pulsed light element 35 are the light emitting diode (LED) 32 as a pulsed light source, and a control element 34 for directing the LED 32. The control element can be embodied by an external processor or controller, or in an embodiment, the LED light source integrally creates pulsed light without any external control signal. In an embodiment, the LED 32 is positioned near the end of the strip-formed element 35, and in a further embodiment, the LED 32 is aligned so that it is capable to emit light around the reflective area marked as 33 without any notable restrictions or shadowing elements. In other words, the LED 32 means the same as an external pulsed light source 32. Also in other words, the external pulsed light source 32 can be placed near the original LED 23, and its light will reflect from the surface of the direct digital detector 22.

Relating to that matter, in an optional solution, a white (or essentially light-colored) reflective tab 33 can be inserted and adhered on the lower surface of the direct digital detector 22. The white reflective tab 33 is placed around the area where the reflection for the incoming light would take place; see the exemplary positioning in Figure 3. Still, it is possible to implement the present invention without the white reflective tab 33; indeed this is possible in the case when the lower surface of the direct digital detector 22 is light-colored on its own.

Now the main functionality of the present invention is described. No matter what the original light source 23 is doing, its outgoing light is blocked totally by the black tab 31. Instead, the added pulsed light element 35 with the LED 32 emits pulsed light. It can be set to emit pulsed light continuously. When the direct digital detector 22 is in its proper use place, its surface section with or without the white reflective tab 33 will reflect the pulsed light towards the photodetector 24 which is the same integral element of the original device. When the X-ray exposure is started for the imaged human tissue (such as the female breast) from the X-ray source, the receiving element is the direct digital detector 22 which receives the X-ray energy propagated through the inspected tissues. The obtained image is transferred by the processor 27 into the memory 28, and if it is desired, the obtained image can also be shown to the doctor or nurse on the screen 29.

Because the added pulsed light element 35 comprising the LED 32 emits only pulsed light, and because the reflected light is thus also pulsed light, the original photodetector 24 may detect a lack of light in a frequent periodical manner, or in more general, a change in the received light intensity level. If the detected light is "1" and lack of light is "0", the photodetector 24 may detect a change to "zero", when the X-ray image has been singularly taken from the human tissue with the direct digital detector 22. Or vice versa, the photodetector 24 may detect a change to "one", when the X-ray image has been singularly taken from the human tissue with the direct digital detector 22. In practice, the photodetector is able to detect a lowering or rising edge of the received light intensity. The processor 27 (as in the integral original device) interprets the newly received zero or newly received one (after the change in signal level) so that it considers that the detecting element has been removed from the bucky. While actually, the direct digital detector does not need to be removed during the usage of the mammogram device, the processor 27 considers after each taken X-image that the detector has been removed and a new detector has been installed. In this way, the processor 27 is "fooled" but this is obtained without touching the original structure of the analog mammogram device, and also without removing the direct digital detector 22. The mammogram device is able to take an X-ray image always when its on, and when the direct digital detector 22 is in its proper place in the bucky.

If the direct digital detector 22 is removed from its proper place, the emitted light from LED 32 will not be reflected to the photodetector 24, nor it can be directly propagated to the photodetector 24 because of their mutual positioning and alignments. In this situation, the photodetector 24 is not able to detect any lowering or rising edge of the received light intensity because its environment lacks light from any source and from any direction. In this situation, the processor 27 prohibits the X-ray source to turn on the X-ray exposure. This functionality ensures that no X-ray exposure is transmitted when a proper detector element is out from its proper place in the bucky.

As a final result, the present invention results in a digitally altered device which is not touched in any other way, for obtaining a continuous possibility to take several mammogram images in an easy manner. Each time an image is taken, it is saved to the memory, and the direct digital detector is able to take another image without having to remove any element from the mammogram apparatus.

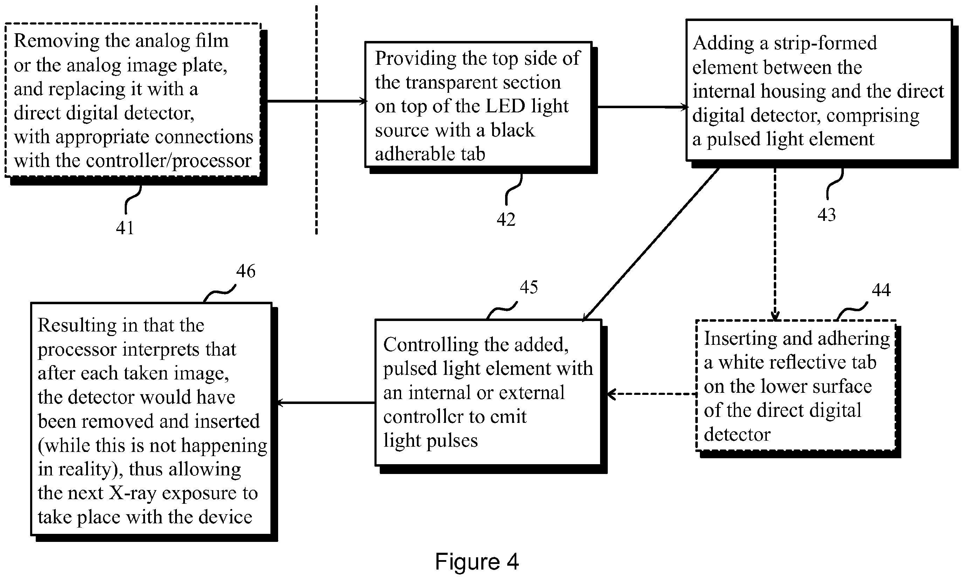

Figure 4 illustrates process steps in a flow chart showing steps for furnishing the traditional analog mammogram device, complemented with a direct digital detector, with further process steps according to the invention for obtaining the desired repeatability function for the taken images. In other words, the result is a digitally transformed mammogram device, which can be used in a continuous basis. The present invention in its own part allows the update of the large quantities of already used analog mammogram devices present already in hospitals and health centres, into a digital form. Such a device update principle is also an ecological solution and much cheaper to the hospitals and health centres than purchasing a totally new digital mammogram device. Also there is no need for transporting large devices anywhere out or in the health centre, because the updating can be done in the local premises right away without a need to move the device.

At the first, optional step which applies to the yet fully analog mammogram device, a changing of the image detector needs to be performed. In other words, at this step, removing the analog film or the analog image plate, and replacing it with a direct digital detector, with appropriate connections with the controller/processor 41.

When we have the analog mammogram device with a direct digital detector 22 in the bucky, the actual process steps of this embodiment of the invention can be applied. At this step, the original LED 23 light within the device housing must be hindered in some way to not illuminate the surface of direct digital detector 22 anymore. This can be obtained by providing the top side of the transparent section on top of the LED light source 23 with a black adherable tab 42. Of course, the effect of the black tab can be achieved by another dark-based coloured tab (i.e. not a transparent one). The tab is adhered onto its place with its own adhering surface, or by other adherable pieces of tape placed around / on top of the tab. The main effect is that no light will propagate out from the original LED's transparent window.

At the next step of this embodiment's configuration, an added light element is provided within the device, without tampering the original design of the device nor opening any part of the housing of the device. In other words, adding a strip-formed element between the internal housing and the direct digital detector, comprising a pulsed light element 43, is the next step of this embodiment.

The next step 44 is an optional step. In there, the optical reflectiveness of the lower surface of the direct digital detector 22 can be increased by adding on top of it a white reflective tab 44. This can also be other fair- or light-coloured tab, which can be adhered on the lower surface of the direct digital detector either self-adhesively or by external taping means, for instance.

Now the transformed arrangement of the digitally modified mammography apparatus is obtained, and the final actual step of the configuration is to control the added, pulsed light element with an internal or external controller to emit light pulses 45. In more detail, the LED 32 being part of the pulsed light element 35 is controlled either with its own controller or by an external control signal provided from 34.

Now the photodetector 24 is able to detect rising and lowering edges of the received light intensity in a continuous manner, and independently of the X-ray exposures and image detections via the direct digital detector 22. In other words, the assembled mammography apparatus arrangement results in that 46 the processor 27 interprets that after each taken image, the direct digital detector 22 would have been removed and inserted (while this is not happening in reality), thus allowing the next X-ray exposure to take place with the apparatus (i.e. the device). This final step 46 of the flow chart of this embodiment therefore shows the intended effect for the mammography apparatus which has been transformed to a digitally operating X-ray imaging tool with an added direct digital detector 22. It can be said that the original analog device design is not touched by any internal intervention, but the principle of the present invention adds intelligently certain additional elements to the X-ray imaging arrangement. As a result, the digitally transformed mammography apparatus "gets fooled" by the presented inventive principle, and it allows the next X-ray exposed image to be taken any time after the previous image has been taken. This results in flexible use of the digitally transformed, updated apparatus. There is no need to remove any parts such as the direct digital detector 22, for the apparatus to function properly after the taken image. This smoothens the use of the device, and ensures that the user cannot break the device, because there are no big elements/parts to be removed from the housing of the apparatus in order to use the device in a continuous manner.

As its clear from the illustration of Figure 4, only steps 42, 43 and 45 are applied in a further embodiment, where only active configuration steps of the apparatus are considered.

In an embodiment, the light source 32 can apply a controlled operational principle where the pulses are created with a constant pulse gap and pulse length. This ensures that there is a rising or a lowering end of the photodetector output signal of 26 relatively soon after any taken X-ray image, if the frequency of the light pulses is not extremely low. In another embodiment, there can be a manual switch or button in connection with the control block 34 of the LED 32. The LED may be in the illumination state in a continuous manner, but the pressing of the manual button or switch can be configured to turn the light of the LED 32 off. In other words, the manual button or switch can be called in general as an external UI control means. In this manner, the operator of the mammography device can press the button whenever the X-ray image has been properly taken. This makes the LED turn off, resulting in a lowering signal edge in the photodetector signal output 26, which the processor interprets as the permission to take a next X-ray exposure image from the patient or from another patient. In this way, there is a bit of manual operation in connection with the X-ray image being taken, but this embodiment allows the LED to regularly emit continuous light. Thus, there are also other types of light sources possible for the light source element 32 than merely a continuously pulsating light.

The embodiments of the present invention are not restricted merely to updating traditionally analog Instrumentarium's mammography apparatuses into digital ones, but the presented embodiments can be applied for digitalization of analog mammography apparatuses by any manufacturer who apply optical means in their devices for checking the change of the analog image plate or film into a new one. One condition is of course that the device structure and dimensions need to enable installment of the direct digital detector into the bucky where the analog detector was placed earlier. Regulatory aspects need to be taken into account, which means that the original design (i.e. parts) of the housing and the X-ray source structure must not be broken. The change of the detector into a digital one does not usually break the original design because the direct digital detector is available in the market in the size applicable in the original bucky.

The advantages of the invention are that the digital transformation for large amounts of analog mammogram devices is easy to perform, and the imaging process can be done without having to perform any tricks to the direct digital detector. Also there is no need to remove the added pulsed light element when the device is in use. The solution is a cost-effective and easy-to-use solution, and it is an ecological solution because the old devices can still be used while the measurements are now fully digital. Also there will be no problems with any regulating bodies of medical equipments anywhere, because the original device of analog mammogram apparatuses are not touched (i.e. no parts are removed or modified). Instead there are added parts, which "fool" the processor, but the modifications by no means endanger the patient safety during the mammographic measurements applying X-rays. Thus, the present invention provides a device, which is also safe to use for both the patient and for the medical professionals. One advantage is that the added parts can also be removed quite easily, because the added strip can be released after the direct digital detector has been removed, and because the white and black tabs are also adhered by detachable tape. The removal of the added parts do not break or stain anything in the original mammogram device.

A further advantage of the presented embodiments is that the realization of the present invention is easy (i.e. the assembly process for the properly functioning digitalized device), and the added parts are very cost effective, i.e. cheap components compared to the actual price tag of the original analog mammography apparatus, or a brand new digital mammography apparatus. The low costs and ease of the digitalization changes ensure that the transformation activities for the devices are well feasible anywhere in the world, where there are old but functioning, analog mammography apparatuses.

The main result, which can also be considered as an advantage, is as follows. The original photodetector 24 is able to detect a lowering or rising edge of the received light signal originated from the external pulsed light source 32 after each taken X-ray exposure image from a patient, thus allowing the mammography apparatus (i.e. by its professional user) to take a next X-ray image from the same patient or another patient without a need for further manoeuvres within the device arrangement itself. This greatly simplifies the general use situation of the digitalized mammography apparatus, without having to purchase a brand new digital mammography apparatus from scratch.

The inventive main concept is an apparatus which has been transformed to a properly functioning digitalized device, but which has been initially manufactured as an analog device. The inventive idea comprises thus also the method of assembly of the invented arrangement. The inventive concept also comprises a computer program and a respective computer program product (i.e. the medium storing the computer program) for controlling the now digitalized mammogram device, which is capable to obtain a plurality of serial mammographic images from the examined patient(s).

In other words, the inventive concept comprises a computer program for controlling operation of a continuously operable digitized mammography apparatus, which is characterized in that the computer program comprises instructions for causing the apparatus to perform at least the following steps:
- controlling an external pulsed light source 32 for emitting pulsed light either automatically or via a manual signal through an external UI control means, wherein the external pulsed light source 32 is placed between an internal housing surface 21 of the apparatus and a surface of the direct digital detector 22,
- exposing an imaged area of a patient with an X-ray beam,
- detecting an X-ray image with a direct digital detector 22 of the digitized mammography apparatus
- saving the detected X-ray image into a memory 28,
- detecting a lowering or rising edge in a signal 26 of a photodetector 24 because of received pulsed light, and because of such detection,
- letting the digitized mammography apparatus to expose an area of the patient or another patient with an X-ray beam and detecting a further X-ray image with the direct digital detector 22.

Furthermore, the inventive concept comprises a non-transitory computer readable medium for controlling operation of a continuously operable digitized mammography apparatus, comprising program instructions for causing the apparatus to perform at least the following steps:
- controlling an external pulsed light source 32 for emitting pulsed light either automatically or via a manual signal through an external UI control means, wherein the external pulsed light source 32 is placed between an internal housing surface 21 of the apparatus and a surface of the direct digital detector 22,
- exposing an imaged area of a patient with an X-ray beam,
- detecting an X-ray image with a direct digital detector 22 of the digitized mammography apparatus
- saving the detected X-ray image into a memory 28,
- detecting a lowering or rising edge in a signal 26 of a photodetector 24 because of received pulsed light, and because of such detection,
- letting the digitized mammography apparatus to expose an area of the patient or another patient with an X-ray beam and detecting a further X-ray image with the direct digital detector 22.

The present invention is not restricted merely to the examples and embodiments mentioned above, but the present invention may vary within the scope of the claims.

## Claims

1. An arrangement for functionally updating a digitalized, initially analog mammography apparatus, wherein the initially analog mammography apparatus comprises a direct digital detector (22) instead of an analog image plate or film, **characterized in that** the arrangement comprises:
- a light blocking element (31) configured on top of a LED light source (23) initially part of the analog mammography apparatus,
- an external pulsed light source (32), which is controlled to emit pulsed light, wherein the external pulsed light source (32) is placed between an internal housing surface (21) of the apparatus and a surface of the direct digital detector (22), wherein
- an original photodetector (24) of the apparatus is able to detect a lowering or rising edge of the received light signal originated from the external pulsed light source (32) after each taken X-ray exposure image from a patient.

2. The arrangement according to claim 1, **characterized in that** the arrangement further comprises:
- the external pulsed light source (32) placed as part of an added pulsed light element (35), which is placable as a strip-formed element between the internal housing surface (21) of the apparatus and the surface of the direct digital detector (22).

3. The arrangement according to claim 2, **characterized in that** the arrangement further comprises:
- the external pulsed light source (32) locating substantially at the far end of the strip.

4. The arrangement according to claim 1, **characterized in that** the arrangement further comprises:
- an external controller (34) controlling the external pulsed light source (32) to emit pulsed light in a predetermined pulse length or frequency.

5. The arrangement according to claim 1, **characterized in that** the arrangement further comprises:
- the external pulsed light source (32) or the added pulsed light element (35) comprising an integrated controller for controlling the external pulsed light source (32) to emit pulsed light in a predetermined pulse length or frequency.

6. The arrangement according to claim 1, **characterized in that** the arrangement further comprises:
- a reflective surface part (33) placeable on top of the direct digital detector (22), which reflective surface part (33) is capable to reflect light originating from the external pulsed light source (32) towards the photodetector (24).

7. The arrangement according to claim 1, **characterized in that** the reflective surface part (33) is a white adherable tab placed approximately in the same area where the initial LED light source (23) light would reflect at during its propagational path to the photodetector (24) during the use of the analog apparatus.

8. A method for furnishing an analog mammography apparatus for resulting as a continuously operable digitized mammography apparatus, **characterized in that** the method comprises the steps of:
- providing a top side of a transparent section of an internal housing (21) on top of the LED light source (23) with a light blocking element (31),
- inserting an added element (35) between the internal housing (21) and a direct digital detector (22), which added element (35) comprises an external pulsed light source (32),
- controlling the added, external pulsed light source (32) with an internal or external (34) controller to emit light pulses in a predetermined pulse length or frequency.

9. The method according to claim 8, **characterized in that**, before the providing step, the method comprises the step of:
- removing an analog image plate or film (12) from a bucky of the analog mammography apparatus, and placing the direct digital detector (22) into the bucky of the analog mammography apparatus.

10. The method according to claim 8, **characterized in that** the method further comprises the step of:
- inserting and adhering a reflective surface part (33) on top of the direct digital detector (22), which reflective surface part (33) is capable to reflect light originating from the external pulsed light source (32) towards the photodetector (24).

11. The method according to claim 8, **characterized in that** the method further comprises the step of:
- turning on the external pulsed light source (32) by the controller, by constantly emitting pulsed light towards the photodetector (24) during the operation of the X-ray imaging.

12. The method according to claim 8, **characterized in that** the method further comprises the step of:
- providing a manual button or switch in connection to the external pulsed light source (32), allowing a professional user of the digitized mammography apparatus to provide a signal through the manual button or switch after each taken X-ray exposure image.

13. A computer program for controlling operation of a continuously operable digitized mammography apparatus, wherein the computer program comprises instructions for causing the apparatus to perform the step of:
- controlling a continuous LED light source (23) to be on all the time by a processor (27);
**characterized in that** the computer program comprises instructions for causing the apparatus to further perform the steps of
- controlling an external pulsed light source (32) for emitting pulsed light either automatically or via a manual signal through a manual button or switch in connection with a control (34) for pulsed light, wherein the external pulsed light source (32) is placed between an internal housing surface (21) of the apparatus and a surface of the direct digital detector (22),
- exposing an imaged area of a patient with an X-ray beam by the processor (27),
- detecting an X-ray image with a direct digital detector (22) of the digitized mammography apparatus by the processor (27),
- saving the detected X-ray image into a memory (28) by the processor (27),
- detecting a lowering or rising edge in a signal (26) of a photodetector (24) because of received pulsed light by the processor (27), and because of such detection,
- letting the digitized mammography apparatus to expose an area of the patient or another patient with an X-ray beam and detecting a further X-ray image with the direct digital detector (22) by the processor (27).

14. A non-transitory computer readable medium for controlling operation of a continuously operable digitized mammography apparatus, comprising program instructions for causing the apparatus to perform at least the following steps:
- controlling a continuous LED light source (23) to be on all the time by a processor (27);
- controlling an external pulsed light source (32) for emitting pulsed light either automatically or via a manual signal through a manual button or switch in connection with a control (34) for pulsed light, wherein the external pulsed light source (32) is placed between an internal housing surface (21) of the apparatus and a surface of the direct digital detector (22),
- exposing an imaged area of a patient with an X-ray beam by the processor (27),
- detecting an X-ray image with a direct digital detector (22) of the digitized mammography apparatus by the processor (27),
- saving the detected X-ray image into a memory (28) by the processor (27),
- detecting a lowering or rising edge in a signal (26) of a photodetector (24) because of received pulsed light by the processor (27), and because of such detection,
- letting the digitized mammography apparatus to expose an area of the patient or another patient with an X-ray beam and detecting a further X-ray image with the direct digital detector (22) by the processor (27).

## Patentansprüche

1. Anordnung zur Funktionsaktualisierung einer digitalisierten, zunächst analogen Mammographievorrichtung, wobei die zunächst analoge Mammographievorrichtung einen direkten digitalen Detektor (22) anstelle einer analogen Bildplatte oder eines analogen Films umfasst, **dadurch gekennzeichnet, dass** die Anordnung Folgendes umfasst:
- ein Lichtblockierelement (31), das auf einer LED-Lichtquelle (23) angeordnet ist, die ursprünglich Teil der analogen Mammographievorrichtung war,
- eine externe gepulste Lichtquelle (32), die zur Emission von gepulstem Licht angesteuert wird, wobei die externe gepulste Lichtquelle (32) zwischen einer inneren Gehäuseoberfläche (21) der Vorrichtung und einer Oberfläche des direkten digitalen Detektors (22) platziert ist, wobei
- ein ursprünglicher Fotodetektor (24) der Vorrichtung in der Lage ist, nach jedem von einem Patienten aufgenommenen Röntgenaufnahmebild eine abfallende oder ansteigende Flanke des empfangenen Lichtsignals zu erkennen, das von der externen gepulsten Lichtquelle (32) stammt.

2. Anordnung nach Anspruch 1, **dadurch gekennzeichnet, dass** die Anordnung ferner Folgendes umfasst:
- die externe gepulste Lichtquelle (32), die als Teil eines zusätzlichen gepulsten Lichtelements (35) angeordnet ist, das als streifenförmiges Element zwischen der inneren Gehäuseoberfläche (21) der Vorrichtung und der Oberfläche des direkten Digitaldetektors (22) platzierbar ist.

3. Anordnung nach Anspruch 2, **dadurch gekennzeichnet, dass** die Anordnung ferner Folgendes umfasst:
- die externe gepulste Lichtquelle (32), die sich im Wesentlichen am anderen Ende des Streifens befindet.

4. Anordnung nach Anspruch 1, **dadurch gekennzeichnet, dass** die Anordnung ferner Folgendes umfasst:
- eine externe Steuerung (34), die die externe gepulste Lichtquelle (32) steuert, um gepulstes Licht mit einer vorgegebenen Pulslänge oder Frequenz auszusenden.

5. Anordnung nach Anspruch 1, **dadurch gekennzeichnet, dass** die Anordnung ferner Folgendes umfasst:
- die externe gepulste Lichtquelle (32) oder das hinzugefügte gepulste Lichtelement (35), umfassend eine integrierte Steuerung zum Steuern der externen gepulsten Lichtquelle (32), um gepulstes Licht mit einer vorbestimmten Pulslänge oder Frequenz auszusenden.

6. Anordnung nach Anspruch 1, **dadurch gekennzeichnet, dass** die Anordnung ferner Folgendes umfasst:
- einen reflektierenden Oberflächenteil (33), das auf dem direkten digitalen Detektor (22) platzierbar ist, wobei der reflektierende Oberflächenteil (33) in der Lage ist, von der externen gepulsten Lichtquelle (32) stammendes Licht in Richtung des Fotodetektors (24) zu reflektieren.

7. Anordnung nach Anspruch 1, **dadurch gekennzeichnet, dass** der reflektierende Oberflächenteil (33) eine etwa im gleichen Bereich angebrachte weiße Klebelasche ist, wobei das Licht der anfänglichen LED-Lichtquelle (23) während seines Ausbreitungswegs zum Fotodetektor (24) während der Verwendung der analogen Vorrichtung reflektiert wird.

8. Verfahren zur Ausstattung einer analogen Mammographievorrichtung als kontinuierlich betriebsfähige digitalisierte Mammographievorrichtung, **dadurch gekennzeichnet, dass** das Verfahren die folgenden Schritte umfasst:
- Bereitstellen einer Oberseite eines transparenten Abschnitts eines Innengehäuses (21) auf der Oberseite der LED-Lichtquelle (23) mit einem Lichtblockierelement (31),
- Einsetzen eines zusätzlichen Elements (35) zwischen dem Innengehäuse (21) und einem direkten digitalen Detektor (22), wobei das zusätzliche Element (35) eine externe gepulste Lichtquelle (32) umfasst,
- Steuern der zusätzlichen, externen gepulsten Lichtquelle (32) mit einer internen oder externen (34) Steuerung, um Lichtimpulse in einer vorgegebenen Impulslänge oder Frequenz auszusenden.

9. Verfahren nach Anspruch 8, **dadurch gekennzeichnet, dass** das Verfahren vor dem Schritt des Bereitstellens den folgenden Schritt umfasst:
- Entfernen einer analogen Bildplatte oder eines analogen Films (12) aus einem Träger der analogen Mammographievorrichtung und Anordnen des direkten digitalen Detektors (22) im Bucky der analogen Mammographievorrichtung.

10. Verfahren nach Anspruch 8, **dadurch gekennzeichnet, dass** das Verfahren ferner den folgenden Schritt umfasst:
- Einsetzen und Aufkleben eines reflektierenden Oberflächenteils (33) auf den direkten digitalen Detektor (22), wobei der reflektierende Oberflächenteil (33) in der Lage ist, von der externen gepulsten Lichtquelle (32) stammendes Licht in Richtung des Fotodetektors (24) zu reflektieren.

11. Verfahren nach Anspruch 8, **dadurch gekennzeichnet, dass** das Verfahren ferner den folgenden Schritt umfasst:
- Einschalten der externen gepulsten Lichtquelle (32) durch die Steuerung, indem während des Betriebs der Röntgenbildgebung ständig gepulstes Licht in Richtung des Fotodetektors (24) emittiert wird.

12. Verfahren nach Anspruch 8, **dadurch gekennzeichnet, dass** das Verfahren ferner den folgenden Schritt umfasst:
- Bereitstellen eines manuellen Knopfs oder Schalters in Verbindung mit der externen gepulsten Lichtquelle (32), der es einem professionellen Benutzer der digitalisierten Mammographievorrichtung ermöglicht, nach jedem aufgenommenen Röntgenbelichtungsbild ein Signal über den manuellen Knopf oder Schalter bereitzustellen.

13. Computerprogramm zur Steuerung des Betriebs einer kontinuierlich betriebsfähigen digitalisierten Mammographievorrichtung, wobei das Computerprogramm Anweisungen umfasst, um die Vorrichtung zu veranlassen, den folgenden Schritt auszuführen:
- Steuern einer kontinuierlichen LED-Lichtquelle (23), so dass sie durch einen Prozessor (27) ständig eingeschaltet ist;
**dadurch gekennzeichnet, dass** das Computerprogramm Anweisungen umfasst, um die Vorrichtung zu veranlassen, ferner die folgenden Schritte auszuführen
- Steuern einer externen gepulsten Lichtquelle (32) zum Aussenden von gepulstem Licht entweder automatisch oder über ein manuelles Signal durch einen manuellen Knopf oder Schalter in Verbindung mit einer Steuerung (34) für gepulstes Licht, wobei die externe gepulste Lichtquelle (32) zwischen einer inneren Gehäuseoberfläche (21) der Vorrichtung und einer Oberfläche des direkten digitalen Detektors (22) platziert ist,
- Belichten eines abgebildeten Bereichs eines Patienten mit einem Röntgenstrahl durch den Prozessor (27),
- Erfassen eines Röntgenbildes mit einem direkten digitalen Detektor (22) der digitalisierten Mammographievorrichtung durch den Prozessor (27),
- Speichern des erfassten Röntgenbildes in einem Speicher (28) durch den Prozessor (27),
- Erkennen einer abfallenden oder ansteigenden Flanke in einem Signal (26) eines Fotodetektors (24) aufgrund des vom Prozessor (27) empfangenen gepulsten Lichts und aufgrund einer solchen Erkennung,
- Belichtenlassen eines Bereichs des Patienten oder eines anderen Patienten mit einem Röntgenstrahl durch die digitalisierte Mammographievorrichtung und Erfassen eines weiteren Röntgenbildes mit dem direkten digitalen Detektor (22) durch den Prozessor (27).

14. Nichtflüchtiges computerlesbares Medium zur Steuerung des Betriebs einer kontinuierlich betriebsfähigen digitalisierten Mammographievorrichtung, die Programmanweisungen umfasst, um die Vorrichtung zu veranlassen, mindestens die folgenden Schritte auszuführen:
- Steuern einer kontinuierlichen LED-Lichtquelle (23), so dass sie durch einen Prozessor (27) ständig eingeschaltet ist;
- Steuern einer externen gepulsten Lichtquelle (32) zum Aussenden von gepulstem Licht entweder automatisch oder über ein manuelles Signal durch einen manuellen Knopf oder Schalter in Verbindung mit einer Steuerung (34) für gepulstes Licht, wobei die externe gepulste Lichtquelle (32) zwischen einer inneren Gehäuseoberfläche (21) der Vorrichtung und einer Oberfläche des direkten digitalen Detektors (22) platziert ist,
- Belichten eines abgebildeten Bereichs eines Patienten mit einem Röntgenstrahl durch den Prozessor (27),
- Erfassen eines Röntgenbildes mit einem direkten digitalen Detektor (22) der digitalisierten Mammographievorrichtung durch den Prozessor (27),
- Speichern des erfassten Röntgenbildes in einem Speicher (28) durch den Prozessor (27),
- Erkennen einer abfallenden oder ansteigenden Flanke in einem Signal (26) eines Fotodetektors (24) aufgrund des vom Prozessor (27) empfangenen gepulsten Lichts und aufgrund einer solchen Erkennung,
- Belichtenlassen eines Bereichs des Patienten oder eines anderen Patienten mit einem Röntgenstrahl durch die digitalisierte Mammographievorrichtung und Erfassen eines weiteren Röntgenbildes mit dem direkten digitalen Detektor (22) durch den Prozessor (27).

## Revendications

1. Agencement pour mettre à jour fonctionnellement un appareil de mammographie initialement analogique numérisé, dans lequel l'appareil de mammographie initialement analogique comprend un détecteur numérique direct (22) au lieu d'une plaque ou d'un film d'image analogique, **caractérisé en ce que** l'agencement comprend :
- un élément de blocage de lumière (31) configuré au-dessus d'une source de lumière à DEL (23) faisant initialement partie de l'appareil de mammographie analogique,
- une source de lumière pulsée externe (32), qui est commandée pour émettre de la lumière pulsée, dans lequel la source de lumière pulsée externe (32) est placée entre une surface de boîtier interne (21) de l'appareil et une surface du détecteur numérique direct (22), dans lequel
- un photodétecteur d'origine (24) de l'appareil est capable de détecter un front descendant ou montant du signal lumineux reçu provenant de la source de lumière pulsée externe (32) après chaque image d'exposition aux rayons X prise d'un patient.

2. Agencement selon la revendication 1, **caractérisé en ce que** l'agencement comprend en outre :
- la source de lumière pulsée externe (32) placée en tant que partie d'un élément de lumière pulsée supplémentaire (35), qui peut être placé sous la forme d'un élément en forme de bande entre la surface de boîtier interne (21) de l'appareil et la surface du détecteur numérique direct (22).

3. Agencement selon la revendication 2, **caractérisé en ce que** l'agencement comprend en outre :
- la source de lumière pulsée externe (32) située sensiblement à l'extrémité éloignée de la bande.

4. Agencement selon la revendication 1, **caractérisé en ce que** l'agencement comprend en outre :
- un contrôleur externe (34) commandant la source de lumière pulsée externe (32) pour émettre une lumière pulsée dans une longueur ou une fréquence d'impulsion prédéterminée.

5. Agencement selon la revendication 1, **caractérisé en ce que** l'agencement comprend en outre :
- la source de lumière pulsée externe (32) ou l'élément de lumière pulsée ajouté (35) comprenant un contrôleur intégré pour commander la source de lumière pulsée externe (32) pour émettre une lumière pulsée dans une longueur ou fréquence d'impulsion prédéterminée.

6. Agencement selon la revendication 1, **caractérisé en ce que** l'agencement comprend en outre :
- une partie de surface réfléchissante (33) pouvant être placée au-dessus du détecteur numérique direct (22), laquelle partie de surface réfléchissante (33) est capable de réfléchir une lumière provenant de la source de lumière pulsée externe (32) vers le photodétecteur (24) .

7. Agencement selon la revendication 1, **caractérisé en ce que** la partie de surface réfléchissante (33) est une languette adhésive blanche placée approximativement dans la même zone dans laquelle la lumière de la source de lumière à DEL initiale (23) se refléterait au cours de son trajet de propagation vers le photodétecteur (24) lors de l'utilisation de l'appareil analogique.

8. Procédé de fourniture d'un appareil de mammographie analogique destiné à devenir un appareil de mammographie numérique fonctionnant en continu, **caractérisé en ce que** le procédé comprend les étapes de :
- fourniture à un côté supérieur d'une section transparente d'un boîtier interne (21) au-dessus de la source de lumière à DEL (23) d'un élément de blocage de lumière (31),
- insertion d'un élément ajouté (35) entre le boîtier interne (21) et un détecteur numérique direct (22), lequel élément ajouté (35) comprend une source de lumière pulsée externe (32),
- commande de la source de lumière pulsée externe ajoutée (32) avec un contrôleur interne ou externe (34) pour émettre des impulsions lumineuses dans une longueur ou fréquence d'impulsion prédéterminée.

9. Procédé selon la revendication 8, **caractérisé en ce que**, avant l'étape de fourniture, le procédé comprend l'étape de :
- retrait d'une plaque ou d'un film d'image analogique (12) d'un plateau de l'appareil de mammographie analogique, et placement du détecteur numérique direct (22) dans le plateau de l'appareil de mammographie analogique.

10. Procédé selon la revendication 8, **caractérisé en ce que** le procédé comprend en outre l'étape de :
- insertion et collage d'une partie de surface réfléchissante (33) au-dessus du détecteur numérique direct (22), laquelle partie de surface réfléchissante (33) est capable de réfléchir une lumière provenant de la source de lumière pulsée externe (32) vers le photodétecteur (24).

11. Procédé selon la revendication 8, **caractérisé en ce que** le procédé comprend en outre l'étape de :
- allumage de la source de lumière pulsée externe (32) par le contrôleur, en émettant constamment de la lumière pulsée vers le photodétecteur (24) pendant le fonctionnement de l'imagerie par rayons X.

12. Procédé selon la revendication 8, **caractérisé en ce que** le procédé comprend en outre l'étape de :
- fourniture d'un bouton ou d'un interrupteur manuel en connexion avec la source de lumière pulsée externe (32), permettant à un utilisateur professionnel de l'appareil de mammographie numérisé de fournir un signal par l'intermédiaire du bouton ou de l'interrupteur manuel après chaque image d'exposition aux rayons X prise.

13. Programme informatique pour commander le fonctionnement d'un appareil de mammographie numérisé fonctionnant en continu, dans lequel le programme informatique comprend des instructions pour amener l'appareil à exécuter l'étape de :
- commande d'une source de lumière à DEL continue (23) pour qu'elle soit allumée en permanence par un processeur (27) ;
**caractérisé en ce que** le programme informatique comprend des instructions pour amener l'appareil à exécuter en outre les étapes de
- commande d'une source de lumière pulsée externe (32) pour émettre de la lumière pulsée soit automatiquement soit via un signal manuel par l'intermédiaire d'un bouton ou d'un interrupteur manuel en connexion avec une commande (34) pour la lumière pulsée, dans lequel la source de lumière pulsée externe (32) est placée entre une surface de boîtier interne (21) de l'appareil et une surface du détecteur numérique direct (22),
- exposition d'une zone imagée d'un patient avec un faisceau de rayons X par le processeur (27),
- détection d'une image radiographique avec un détecteur numérique direct (22) de l'appareil de mammographie numérisé par le processeur (27),
- enregistrement de l'image radiographique détectée dans une mémoire (28) par le processeur (27),
- détection d'un front descendant ou montant dans un signal (26) d'un photodétecteur (24) en raison d'une lumière pulsée reçue par le processeur (27), et en raison d'une telle détection,
- le fait de laisser l'appareil de mammographie numérisé exposer une zone du patient ou d'un autre patient avec un faisceau de rayons X et de détecter une autre image radiographique avec le détecteur numérique direct (22) par le processeur (27).

14. Support lisible par ordinateur non transitoire pour commander le fonctionnement d'un appareil de mammographie numérisé fonctionnant en continu, comprenant des instructions de programme pour amener l'appareil à exécuter au moins les étapes suivantes :
- commande d'une source de lumière à DEL continue (23) pour qu'elle soit allumée en permanence par un processeur (27) ;
- commande d'une source de lumière pulsée externe (32) pour émettre de la lumière pulsée soit automatiquement soit via un signal manuel par l'intermédiaire d'un bouton ou d'un interrupteur manuel en connexion avec une commande (34) pour la lumière pulsée, dans lequel la source de lumière pulsée externe (32) est placée entre une surface de boîtier interne (21) de l'appareil et une surface du détecteur numérique direct (22),
- exposition d'une zone imagée d'un patient avec un faisceau de rayons X par le processeur (27),
- détection d'une image radiographique avec un détecteur numérique direct (22) de l'appareil de mammographie numérisé par le processeur (27),
- enregistrement de l'image radiographique détectée dans une mémoire (28) par le processeur (27),
- détection d'un front descendant ou montant dans un signal (26) d'un photodétecteur (24) en raison d'une lumière pulsée reçue par le processeur (27), et en raison d'une telle détection,
- le fait de laisser l'appareil de mammographie numérisé exposer une zone du patient ou d'un autre patient avec un faisceau de rayons X et de détecter une autre image radiographique avec le détecteur numérique direct (22) par le processeur (27).
